Europäisches Patentamt

European Patent Office

Office européen des brevets

(19)

(11) **EP 0 602 078 B1**

(12) **EUROPÄISCHE PATENTSCHRIFT**

(45) Veröffentlichungstag und Bekanntmachung des Hinweises auf die Patenterteilung:
**07.02.1996 Patentblatt 1996/06**

(21) Anmeldenummer: **92917679.0**

(22) Anmeldetag: **24.08.1992**

(51) Int. Cl.$^6$: **A61K 7/08**, A61K 7/06

(86) Internationale Anmeldenummer: **PCT/EP92/01940**

(87) Internationale Veröffentlichungsnummer: **WO 93/04662 (18.03.1993 Gazette 1993/08)**

(54) **MILDE REINIGUNGSMITTEL**

MILD CLEANING AGENTS

DETERGENTS DOUX

(84) Benannte Vertragsstaaten:
**AT BE CH DE DK ES FR GB GR IE IT LI NL SE**

(30) Priorität: **02.09.1991 DE 4129124**

(43) Veröffentlichungstag der Anmeldung:
**22.06.1994 Patentblatt 1994/25**

(73) Patentinhaber: **Henkel Kommanditgesellschaft auf Aktien**
**D-40191 Düsseldorf (DE)**

(72) Erfinder:
- **MÜLLER, Reinhard**
  **D-5140 Erkelenz 7 (DE)**
- **SEIDEL, Kurt**
  **D-4000 Düsseldorf 13 (DE)**
- **HOLLENBERG, Detlef**
  **D-4006 Erkrath 2 (DE)**
- **PATTEN, Anja**
  **D-4019 Monheim (DE)**

(56) Entgegenhaltungen:
**EP-A- 0 070 077**      **EP-A- 0 358 216**
**WO-A-91/13961**      **DE-A- 3 503 618**

**Beschreibung**

Die Erfindung betrifft milde Reinigungsmittel, insbesondere Mittel zum Waschen oder Spülen von Haaren.

Zur Erzielung einer reinigenden Wirkung enthalten wäßrige Reinigungsmittel üblicherweise oberflächenaktive Verbindungen, wodurch in der Regel die Hautbelastung des Mittels erhöht wird. Dies gilt besonders für die wichtige Klasse der Aniontenside.

Diese erhöhte Hautbelastung sollte im Bereich der Körperreinigungsmittel vermieden werden. Insbesondere bei Produkten, die für eine häufige Anwendung konzipiert sind, zur Reinigung des Intimbereiches dienen oder mit Schleimhäuten in Berührung kommen, ist eine gute Hautverträglichkeit wichtig. Es besteht daher ein ständiger Bedarf an milden wäßrigen Reinigungsmitteln mit guten Schaumvermögen.

Von besonderem Interesse auf dem Gebiet der wäßrigen Reinigungsmittel sind Mittel zum Waschen oder Spülen von Haaren, zum Beispiel Shampoos und abspülbare Haarnachbehandlungsmittel. Dabei besteht insbesondere ein ständiger Bedarf nach solchen Mitteln, die eine verbesserte Wirkung hinsichtlich Fülle und Frisierbarkeit der Haare aufweisen.

Das Haupthaar weist nach dem Waschen oft einen kosmetisch unbefriedigenden Zustand auf. Es fühlt sich stumpf an, ist im nassen Zustand nur schwer zu kämmen und neigt im trockenen Zustand zur statischen Aufladung wodurch das Kämmen erschwert und der Sitz des gekämmten Haares gestört wird.

Bekannt ist die Verwendung zwitterionischer Polymerer, die anionische Gruppen, in der Regel Carboxylgruppen, und quartäre Ammoniumgruppen im Molekül enthalten, in Haarbehandlungsmitteln. Beispielsweise beschreibt die DE-OS-21 50 557 die Verwendung von Polymerisaten zwitterionischer Monomerer in Haarfestlegemittel. Zwitterionische Polare zeigen jedoch insbesondere in Formulierungen mit anionischen Tensiden den Nachteil, daß die haaravivierenden und haarfestigenden Eigenschaften im Laufe längerer Lagerzeit allmählich verloren gehen.

Aus der deutschen Offenlegungsschrift DE-OS-33 26 230 sind wäßrige Zubereitungen zum Waschen und Spülen der Haare bekannt, die infolge ihrers Gehalts an speziellen Polyaldehydocarbonsäuren die Fülle und Frisierbarkeit der Haare verbessern sollen.

Aus der DE-A-3503618 sind Mittel zum Waschen und Spülen von Haaren bekannt, bei denen die haarkosmetische Wirkung der enthaltenen ionischen Polaren durch spezielle Di- und Tricarbonsäuren verbessert wird. In einem Beispiel wird eine Kombination aus anionischem Polymer und Alkylglykosid genannt.

Die EP-A-0070077 beschreibt schäumende Tensidmischungen aus Alkylglykosiden und anionischen Tensiden, die beispielsweise in Haarwaschmitteln verwendet werden können.

Die EP-A-0358216 beschreibt Tensidmischungen aus Alkylglykosiden und Sulfosuccinaten, die sich durch exzellentes Schaumvermögen auszeichnen. Aufgrund ihres geringen Irritationspotentials sind diese Tensidmischungen für Haar- und Hautbehandlungsmittel geeignet.

Es ist weiterhin bekannt, nach dem Waschen oder Shampoonieren des Haares konditionierende Präparate, meist auf Basis kationischer Tenside, einwirken zu lassen oder den Haarwaschmitteln konditionierende Stoffe zuzusetzen, um gleichzeitig mit der Haarwäsche einen gewissen konditionierenden Effekt zu erzielen. Solche Substanzen sind zum Beispiel kationische Polymere, zum Beispiel kationische Cellulosederivate. Aus der europäischen Patentanmeldung EP 337 354 sind auch Mittel bekannt, die eine Kombination von Alkylglykosiden und einem kationischen Polymer enthalten.

Mit diesen Hilfsmitteln wird zwar eine befriedigende Verbesserung der Naßkämmbarkeit und, zum Beispiel mit kationischen Tensiden, auch eine Verringerung der statischen Aufladung erzielt. Diese Effekte gehen jedoch praktisch immer mit einer zu starken Glättung des trockenen Haares einher. Dadurch entsteht der Nachteil, daß das Haar wenig Fülle und die Frisur keinen Halt aufweist. Die Glätte des Haares ist um so ausgeprägter, je niedriger der Kämmwiderstand des trockenen Haares ist.

Es bestand daher die Aufgabe wäßrige Reinigungsmitteln, insbesondere Mittel zum Waschen und Spülen von Haaren, aufzufinden, welche bei gleichzeitig gutem Schaumvermögen und geringer Hautbelastung die Glätte des trockenen Haares merklich verringern, ohne eine Klebrigkeit des Haares zu verursachen und ohne die Naßkämmbarkeit zu beeinträchtigen.

Es wurde nun überraschend gefunden, daß Fülle und Frisierbarkeit der Haare im Vergleich zum bekannten Stand der Technik deutlich verbessert werden, wenn das wäßrige Reingungsmittel 1 bis 50 Gew.-% eines Aniontensids, 0,5 bis 10 Gew.-% eines Alkylglykosids und 0,1 bis 5 Gew.-% eines anionischen Polymers enthält.

Gegenstand der Erfindung sind daher wäßrige Reinigungsmittel enthaltend

(A) 1 bis 50 Gew.-% eines oder mehrerer Aniontenside, die 1 oder 2 lipophile Reste mit 1 bis 22 C-Atomen und einen polaren Rest ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,

(B) 0,5 bis 10 Gew.-% eines oder mehrerer Alkylglycoside der allgemeinen Formel $R(G)_x$ worin R einen linearen, gesättigten Alkylrest mit 8 bis 22 C-Atomen und $(G)_x$ ein Glycosid- oder Oligoglycosidrest mit einem Oligomerisationsgrad x von 1 bis 4 ist,

(C) 0,1 bis 5 Gew.-% eines anionischen Polymers,

(D) 35 bis 98,4 Gew.-% Wasser.

wobei die Summe der Komponenten (B) und (C) nicht größer als der Gehalt an Komponente (A) ist.

Unter Polyoxyalkylenrest wird dabei eine Gruppe verstanden, die aus Oxyethylen-Einheiten -[CH$_2$-CH$_2$-O]- oder aus Oxypropylen-Einheiten -[CH(CH$_3$)-CH$_2$-O]- aufgebaut ist. Die mittlere Anzahl der Oxyethylen- bzw. Oxypropylen-Einheiten wird dabei als mittlerer Alkoxylierungsgrad bezeichnet.

Die **Aniontenside (A)** werden erfindungsgemäß bevorzugt ausgewählt aus der Gruppe der Alkyl- und Dialkylethersulfate, Ethercarbonsäuren, Sulfobernsteinsäurehalbester, Fettalkoholethercitrate, Fettalkoholethertartrate, Acylsarkoside, Acyltauride und der Sulfonate von ungesättigten Fettsäuren.

Die Gegenionen der Carboxylat-, Sulfat oder Sulfonatreste werden bevorzugt aus der Gruppe der Alkali- und Erdalkalimetalle, Aluminium, Ammonium sowie Alkyl- oder Alkylolammoniumgruppen mit 1 bis 4 C-Atomen in jeder Alkyl- oder Alkylolgruppe gewählt. Ganz besonders geeignet ist die Gruppe der Alkalimetalle.

Die chemischen Strukturen sowie die grundlegenden Tensideigenschaften der meisten dieser Aniontenside gehören mittlerweile zum Lehrbuchwissen und bedürfen daher keiner weiteren Erläuterung. Unter **Dialkylethersulfaten** sind Verbindungen zu verstehen wie sie in der Europäischen Patentameldung EP 299 370 beschrieben sind. Aus dieser Schrift können Einzelheiten des Herstellungsverfahrens und der Eigenschaften dieser Verbindungen entnommen werden. Unter **Fettalkoholethertartraten** sind Monoestersalze der Weinsäure, unter **Fettalkoholethercitraten** Mono- und/oder Diestersalze der Zitronensäure mit Anlagerungsprodukten von Ethylenoxid und/oder Propylenoxid an Fettalkohole zu verstehen. Unter **Sulfonaten von ungesättigten Fettsäuren** sind Sulfonierungsprodukte von Fettsäuren mit 12 bis 22 C-Atomen und 1 bis 6 Doppelbindungen zu verstehen. Derartige Produkte sind literaturbekannt und beispielsweise durch Umsetzung dieser Fettsäuren mit gasförmigem Schwefeltrioxid zugänglich. Am Beispiel der Ölsäure können Einzelheiten des Herstellungsverfahrens der deutschen Patentanmeldung DE 39 26 344 entnommen werden.

Bei Aniontensiden, die einen Polyoxyalkylenrest enthalten, gilt bezüglich des Alkoxylierungsgrades ganz allgemein, daß bei Alkoxylierungsreaktionen wie beispielsweise der Anlagerung von x mol Ethylenoxid an 1 mol Fettalkohol nach den bekannten Ethoxylierungsverfahren kein einheitliches Addukt, sondern ein Gemisch aus Restmengen freien Fettalkohols und einer Reihe homologer (oligomerer) Anlagerungsprodukte von 1, 2, 3, ... x, x+1, x+2 ...usw. Molekülen Ethylenoxid je Molekül Fettalkohol erhalten wird. Der mittlere Ethoxylierungsgrad (x) wird dabei definiert durch die Ausgangsmengen an Fettalkohol und Ethylenoxid. Die Verteilungskurve des Homologengemisches weist in der Regel ein Maximum im Bereich zwischen x-3 und x+3 auf. Nähere Informationen hierzu können beispielsweise der Zeitschrift Soap/Cosmetics/Chemical Specialities, Heft Januar 1988, S. 34, entnommen werden. Neben den üblichen aus dem Stand der Technik bekannten Alkoxylierungskatalysatoren wie Natriummethanolat lassen sich dabei aber auch solche Katalysatoren verwenden, die zu Produkten mit sogenannter eingeengter Homologenverteilung führen, vergl. z.B. Seifen-Öle-Fette-Wachse 1990 (116) 60.

In einer bevorzugten Ausführungsform der Erfindung beträgt der Anteil der anionischen Tenside 5 bis 30 Gew.-%.

**Alkylglycoside (B)** der allgemeinen Formel R-(G)$_x$ sind seit langem bekannte oberflächenaktive Stoffe, die aus Zuckern und aliphatischen, primären Alkoholen mit 8 - 22 C-Atomen unter Acetalisierung herstellbar sind. Als Zuckerkomponenten (Glycosen) kommen bevorzugt Glucose, daneben aber auch Fructose, Mannose, Galactose, Telose, Gulose, Allose, Altrose, Idose, Arabinose, Xylose, Lyxose, Libose und Gemische davon in Frage.

Bevorzugt wegen der leichten Zugänglichkeit und der guten Anwendungseigenschaften sind die Acetalisierungsprodukte der Glucose mit Fettalkoholen R-OH, die z. B. aus natürlichen Fetten und Ölen nach bekannten Verfahren erhältlich sind, insbesondere mit linearen, primären, gesättigten und ungesättigten Fettalkoholen mit 8 bis 22 C-Atomen.

Alkylglycoside, ihre Herstellung und Verwendung als oberflächenaktive Stoffe sind beispielsweise aus US-A-3.839.318, US-A-3.707.535, US-A-3.547.828, DE-A-1943689, DE-A-2036472, DE-A-3001064 sowie EP-A-77167 bekannt. Bezüglich des Glycosidrestes - (G)$_x$ gilt, daß sowohl Monoglycoside (x = 1), bei denen ein Zuckerrest glycosidisch mit dem Fettalkohol verbunden ist, als auch oligomere Glycoside mit einem Oligomerisationsgrad x = 2 bis 4 geeignet sind. In der Regel liegen Gemische von Mono- und Oligoglycosiden vor.

Bevorzugt geeignet sind solche Alkylglycoside (B), in welchen R eine Alkylgruppe mit 8 bis 22 C-Atomen und (G)$_x$ ein Glycosid- oder Oligoglycosidrest mit einem Oligomerisationsgrad x = 1 bis 4 ist. Ganz besonders bevorzugt ist R eine Alkylgruppe mit 10 bis 16 C-Atomen, und (G)$_x$, der Rest eines Gemisches von Glucosid und Oliglucosiden mit einem mittleren Oligomerisationsgrad von 1 bis 1,5.

In eine bevorzugten Ausführungsform der Erfindung beträgt der Anteil des Alkylglykosids 1 bis 5 Gew.-%.

Die Wahl der **anionischen Polymeren (C)** unterliegt an sich keinen besonderen Einschränkungen. Als besonders geeignet haben sich jedoch Polyaldehydocarbonsäuren mit einem mittleren Molekulargewicht von 600 bis 10000 und einem Gehalt von 5 bis 9, vorzugsweise 7 bis 9 Carboxylgruppen und 1 bis 5, vorzugsweise 1 bis 3 Aldehydgruppen pro 10 Monomereinheiten erwiesen. Diese Produkte werden in Form ihrer wasserlöslichen Salze, insbesondere Alkalimetallsalze eingesetzt. Solche Polyaldehydocarbonsäuren sind bekannte Handelsprodukte. Sie werden zum Beispiel durch eine oxidative Homopolymerisation von Acrolein oder auch durch oxidative Copolymerisation von Acrolein und Acrylsäure hergestellt und zum Beispiel von der Firma Degussa vertrieben.

In einer bevorzugten Ausführungsform der Erfindung beträgt der Anteil des anionischen Polymers 0,5 bis 3 Gew.-%.

Weiterhin können die erfindungsgemäßen Mittel neben den Aniontensiden (A) zusätzlich 0,5-20 Gew.-%, insbesondere 1-10 Gew.-%, an ampholytischen und/oder zwitterionischen Tensiden enthalten.

Unter **ampholytischen Tensiden** werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -SO$_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarkosine, 2-Alkylaminopropionsäuren und Alkylaminoessigsäuren mit jeweils etwa 8 bis 18 C-Atomen in der Alkylgruppe.

Als **zwitterionische Tenside** werden solche oberflächenaktiven Verbindungen bezeichnet, die im Molekül mindestens eine quartäre **Ammoniumgruppe** und mindestens eine -COO$^{(-)}$- oder -SO$_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinat, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Die hautschonenden Eigenschaften der erfindungsgemäßen Mittel kommen besonders dann zur Geltung, wenn diese so formuliert werden, daß sie einen pH-Wert in der Nähe des Neutralpunktes der Haut aufweisen. Mittel mit pH-Werten im Bereich von 4,0 - 7,5, insbesondere von 4,5 - 7,0, sind daher bevorzugt.

Die erfindungsgemäßen Mittel können darüber hinaus **anorganische Elektrolytsalze (E)** enthalten. Dazu eignen sich alle wasserlöslichen Alkali-, Ammonium- und Erdalkalisalze, z. B. die Fluoride, Chloride, Bromide, Sulfate, Phosphate und Nitrate und Hydrogencarbonate, soweit sie in einer Menge von wenigstens 1 Gew.-% bei 20°C in Wasser löslich sind. Bevorzugt werden die Chloride oder Sulfate eines Alkalimetalls, des Ammoniums oder des Magnesiums verwendet; besonders bevorzugt sind Natriumchlorid und Magnesiumchlorid. Bevorzugt beträgt die Menge des Elektrolytsalzes 0,1 bis 10 Gew.-%.

Die erfindungsgemäßen Mittel können in einer Vielzahl von reinigenden Konsumentenprodukten wie Haarshampoos, Schaumbädern, Duschbädern, flüssigen Seifen und manuellen Geschirrspülmitteln Verwendung finden. Insbesondere eignen sie sich für milde Haarshampoos mit verbesserter Fülle und Frisierbarkeit der Haare.

Diese Produkte enthalten neben Tensiden oder Tensidkombinationen üblicherweise Bestandteile wie Emulgatoren, Ölkomponenten, Lösungsvermittler, Verdickungsmittel, Überfettungsmittel, biogene Wirkstoffe, Filmbildner, Duftstoffe, Farbstoffe, Perlglanzmittel, Schaumstabilisatoren, Konservierungsmittel und pH-Regulatoren. Die erfindungsgemäßen Mittel können daher zusätzliche Komponenten und Hilfsstoffe enthalten, wie sie aus dem Stand der Technik bekannt sind. Die wichtigsten sind:

**Tenside/Emulgatoren,** z.B. anionaktive Tenside mit Carboxylat-, Sulfonat-, Sulfat- oder Phosphatgruppen wie Seifen, Alkyl- und Arylethersulfate, Fettamine, quartäre Ammonium- und Pyridiniumverbindungen, nichtionische Emulgatoren wie Ethylenoxidaddukte an Alkohole, Carbonsäuren, Partialglyceride und Sorbitanester, amphotere und zwitterionische Emulgatoren wie Imidazolinderivate, Betaine oder Sulfobetaine sowie z.B. Fettsäureester und Sorbitanfettsäureester (vergl. z.B. W. Umbach [Hrsg.], "Kosmetik - Entwicklung, Herstellung und Anwendung kosmetischer Mittel", S.86-87, Stuttgart 1988).

**Ölkomponenten,** z.B. Substanzen wie Paraffinöl, Pflanzenöle, Fettsäureester, Squalan und 2-Octyldodecanol; als Fette und Wachse beispielsweise Walrat, Bienenwachs, Montanwachs, Paraffin und Cetyl-stearylalkohol.

**Lösungsvermittler,** z.B. niedrige ein- oder mehrwertige Alkohole wie Ethanol, Isopropanol, Ethylenglykol, Propylenglykol, Glycerin, 1,3-Butylenglykol und Diethylenglykol.

**Verdickungsmittel,** z.B. Polysaccharide, insbesondere Xanthan-Gum, Guar-Gum, Agar-Agar, Alginate und Tylosen, sowie Carboxymethylcellulose und Hydroxyethylcellulose, ferner höhermolekulare Polyethylenglykolmono- und -diester von Fettsäuren, Polyacrylate, Polyvinylalkohol und Polyvinylpyrrolidon.

**Überfettungsmittel,** z.B. polyethoxylierte Lanolinderivate, Lecithinderivate und Fettsäurealkanolamide, wobei die letzteren gleichzeitig auch als Schaumstabilisatoren dienen.

**Biogenen Wirkstoffe** wie Pflanzenextrakte, Eiweißabbauprodukte und Vitaminkomplexe.

**Filmbildner** wie Polyvinylpyrrolidon, Vinylpyrrolidon-Vinylacetat-Copolymerisate, Polymere der Acrylsäurereihe, quaternäre Cellulose-Derivate und ähnliche Verbindungen.

**Feuchthaltemittel,** z.B. Glycerin, Polyglycerine, Sorbit, 1,2-Propandiol, 1,2,3-Butan-triol, Polyethylenglykole, Glucose, Mannit, Xylit, Pyrollidon-Carbonsäure-Salze (PCA), Aminosäuren, Milchsäure.

**Antimikrobiell wirksame Stoffe** als Konservierungsmittel, z.B. Benzoesäure, Salicylsäure, Sorbinsäure, sowie deren Ester und Salze sowie die in der Anlage zur Kosmetikverordnung aufgeführten Substanzen.

**Perlglanzmittel** wie Glykoldistearinsäureester, Ethylenglykoldistearat oder Fettsäuremonoglykolester.

**Duftstoffe,** z.B. natürliche Riechstoffe, die durch Destillation, Extraktion oder Pressung aus Pflanzen gewonnen werden sowie synthetisch hergestellte Riechstoffe (vergl. z.B. H.Aebi, E.Baumgartner, H.P.Fiedler, G.Ohloff, "Kosmetika, Riechstoffe und Lebensmittelzusatzstoffe", Stuttgart 1978).

**Antioxidantien,** z.B. Tocopherole, Lecithin, Guajakol, Butylkresol, 4-Methyl-2,6-di-tert.-butyl-phenol (BHT), 4-Methoxy-2(3)tert.-butylphenol (BHA)

**Farbstoffe,** wie sie z.B. von der Farbstoff-Kommission der Deutschen Forschungsgemeinschaft für Kosmetika zusammengestellt sind ("Färbemittel für Kosmetika" Mitteilung 3, Wiesbaden 1968). Die Farbstoffe werden üblicherweise in Konzentrationen von 0,001 bis 0,1 Gew.-%, bezogen auf die gesamte Mischung, eingesetzt.

**pH-Regulatoren:** Weitere Komponenten der erfindungsgemäßen Mittel sind gewünschtenfalls Substanzen, die der Einstellung des pH-Wertes der Mittel dienen.

Die Gesamtmenge der Hilfsstoffe beträgt 0 - 20 Gew.-%, vorzugsweise 0 - 10 Gew.-%.

Zur Herstellung der erfindungsgemäßen Mittel wird das Alkylglycosid (B) bei 60 bis 80 °C zu einer wäßrigen Phase gegeben, die die Aniontenside (A), das anionische Polymer (C) und das Elektrolytsalz (D) enthält. Diese Mischungen werden gerührt und dann auf Normaltemperatur (20 bis 40 °C) abgekühlt. Wegen der Wasserlöslichkeit der Alkylglycoside, insbesondere solcher mit Alkylresten von 12 bis 16 C-Atomen, können die erfindungsgemäßen Mittel vorteilhaft auch auf kaltem Wege hergestellt werden. Dabei werden die Komponenten in einfacher Weise bei Normaltemperatur zusammengerührt.

Die nachfolgenden Beispiele dienen der Erläuterung der Erfindung und sind nicht einschränkend aufzufassen.

## Beispiele

### 1. Allgemeines

#### 1.1. Abkürzungen

In den Kopfzeilen der Tabellen 1 bis 5 sind die Beispiele mit **B1** bis **B5**, die Vergleiche mit **V1** bis **V10** kenntlich gemacht.

#### 1.2. Verwendete Substanzen

#### 1.2.1. Tenside

**N25:** Wäßrige Lösung von Natriumlaurylethersulfat; Aktivsubstanzgehalt: 28 Gew.-% ("Texapon[(R)] N25"; Fa. Henkel/Düsseldorf)

**Dehyton K:** Wäßrige Lösung eines Fettsäureamid-Derivats mit Betainstruktur der Formel R-CONH-$(CH_2)_3$-$N^+(CH_3)_2$-$CH_2$-$COO^-$ ; CTFA-Bezeichnung: Cocamidopropyl Betaine; Aktivsubstanzgehalt: 30 Gew.-%; NaCl-Gehalt: 5 Gew.-% (Fa. Henkel/Düsseldorf)

**AKYP:** Wäßrige Lösung eines Ethercarbonsäuresalzes der Formel $C_{12/14}$-$(O-CH_2-CH_2)_{10}$-$OCH_2$-COONa, Aktivsubstanzgehalt: 22 Gew. % ("Akypo[(R)]-Soft 100 NV"; Fa. Chemy-Y)

**APG600:** $C_{12/14}$-Fettalkoholglucosid mit einem Oligomerisationsgrad von 1,45 (Fa. Henkel/Düsseldorf).

#### 1.2.2. Sonstige Stoffe

**Nutrilan I:** Eiweiß-Hydrolysat; CTFA-Bezeichnung: hydrolyzed-animal-collagen ("Nutrilan[(R)] I"; Fa. Grünau/Illertissen)

**POC:** Wäßrige Lösung von Poly(aldehydocarbonsäure)-Natrium-Salz; Aktivsubstanzgehalt: 40 Gew.-%; mittleres Molekulargewicht: 5000 ("POC-HS-5060"; Fa. Degussa)

### 2. Bestimmung der Trockenkämmbarkeit

Den Untersuchungen zur Kämmbarkeit wurde die Methode gemäß J. Soc. Cosm. Chem. 1973 [24] 782 zugrundegelegt.

Die Trockenkämmarbeit wurde an braunem Haar (Alkinco #6634, Strähnenlänge 12 cm, Strähnenmasse 1 g) untersucht. Es handelte sich um leicht vorgeschädigte (blondierte) Haare, wie sie etwa beim durchschnittlichen Anwender zu erwarten sind. Nach der Nullmessung wurden die Strähnen mit 100 ml der zu prüfenden Rezeptur getränkt. Nach 5 Minuten Einwirkzeit wurden die Strähnen 1 Minute unter fließendem Wasser (1 l/min, 38 °C) ausgespült. Die Strähnen wurden anschließend 12 Stunden bei 30 °C und einer relativen Luftfeuchtigkeit von 20% getrocknet und dann erneut vermessen und das Ergebnis mit der Nullmessung verglichen. Die Ergebnisse der Trockenkämmbarkeitsversuche sind in der Tabelle 1 zusammengestellt.

Tabelle 1

| Trockenkämmbarkeit[1] | | | |
|---|---|---|---|
| Substanz | V1 | V2 | B1 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| POC | - | 1,0 | 1,0 |
| APG 600 | - | - | 2,0 |
| Wasser | ad 100 | ad 100 | ad 100 |
| TK (%)[2] | 95 | 122 | 146 |

[1] Die Angaben im oberen Block der Tabelle beziehen sich auf Gew.-% Aktivsubstanz. Die Angaben im unteren Block der Tabelle beziehen sich auf den experimentell ermittelten Wert der Trockenkämmbarkeit TK im Vergleich zur Nullmessung.
[2] Die statistische Sicherheit der TK-Werte betrug 99,99%.

Das erfindungsgemäße Beispiel B1 weist eine im Vergleich zu V1 und V2 deutlich höhere Trockenkämmbarkeit auf. Da TK ein Maß für den Halt der Frisur ist, ist für B1 eine Verbesserung des Frisurenhalts nachgewiesen.

## 3. Bestimmung der Naßkämmbarkeit

Den Untersuchungen zur Kämmbarkeit wurde die Methode gemäß J. Soc. Cosm. Chem. 1973 [24] 782 zugrundegelegt.
Die Naßkämmarbeit wurde an braunem Haar (Alkinco #6634, Strähnenlänge 12 cm, Strähnenmasse 1 g) untersucht. Es handelte sich um leicht vorgeschädigte (blondierte) Haare, wie sie etwa beim durchschnittlichen Anwender zu erwarten sind. Nach der Nullmessung wurden die Strähnen mit 100 ml der zu prüfenden Rezeptur getränkt. Nach 5 Minuten Einwirkzeit wurden die Strähnen 1 Minute unter fließendem Wasser (1 l/min, 38 °C) ausgespült. Die Strähnen wurden erneut vermessen und das Ergebnis mit der Nullmessung verglichen. Die Ergebnisse der Naßkämmbarkeitsversuche sind in der Tabelle 2 zusammengestellt.

Tabelle 2

| Naßkämmbarkeit[1] | | | |
|---|---|---|---|
| Substanz | V3 | V4 | B2 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| POC | - | 1,0 | 1,0 |
| APG 600 | - | - | 2,0 |
| Wasser | ad 100 | ad 100 | ad 100 |
| NK (%)[2] | 94 | 92 | 80 |

[1] Die Angaben im oberen Block der Tabelle beziehen sich auf Gew.-% Aktivsubstanz. Die Angaben im unteren Block der Tabelle beziehen sich auf den experimentell ermittelten Wert der Naßkämmbarkeit NK im Vergleich zu Nullmessung.
[2] Die statistische Sicherheit der NK-Werte betrug 99,99%.

Das erfindungsgemäße Beispiel B2 zeigt einen im Vergleich zu V3 und V4 deutlich geringeren Wert der Naßkämmbarkeit. Da geringere Werte von NK eine bessere Kämmbarkeit von nassem Haar bedeuten, ist für B2 eine Verbesserung der Naßkämmbarkeit gezeigt.

**4. Elektrostatische Aufladung**

Zur Bestimmung der elektrostatischen Aufladung beim Kämmen wurde eine Haarsträhne innerhalb eines doppelten Faraday-Käfigs aufgehängt. Zwischen innerem und äußerem Käfig bestand keine leitende Verbindung. Der äußere Käfig war mit dem Nullpotential (Erde) verbunden. Ein Voltmeter maß Potentialdifferenzen zwischen innerem und äußerem Käfig. Potentialdifferenzen entstehen immer dann, wenn aufgrund von Reibungsarbeiten beim Kämmen Ladungen von den Oberflächen der Haarfasern abgezogen werden und sich auf der Kammoberfläche sammeln. Das elektrische Feld und die im Vergleich zum Kamm entgegengesetzt aufgeladene Strähne tritt mit dem inneren Faraday-Käfig in Wechselwirkung. Dieser lädt sich auf der Innenseite entgegengesetzt auf (Influenz). Die Ladungsverschiebung gegenüber dem äußeren Käfig wurde am Voltmeter registriert und war ein direktes Maß für die triboelektrische Aufladung der gekämmten Haarsträhne.

Tabelle 3

| Elektrostatische Aufladung[1] | | | |
|---|---|---|---|
| Substanz | V5 | V6 | B3 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| POC | - | 1,0 | 1,0 |
| APG 600 | - | - | 2,0 |
| Wasser | ad 100 | ad 100 | ad 100 |
| EA (%) | 92 | 90 | 73 |

[1] Die Angaben im oberen Block der Tabelle beziehen sich auf Gew.-% Aktivsubstanz. Die Angaben im unteren Block der Tabelle beziehen sich auf den experimentell ermittelten Wert der elektrostatischen Aufladung EA im Vergleich zu Nullmessung.

Das erfindungsgemäße Beispiel B3 zeigt einen im Vergleich zu V5 und V6 wesentlich geringeren Wert der elektrostatischen Aufladung. Geringere Werte für EA bedeuten eine verminderte Tendenz der Haare, sich gegenseitig abzustoßen und auseinanderzustreben ("fly-away-Effekt").

## 5. Bestimmung des Schaumverhaltens

Das Schaumverhalten der Reinigungsmittel wurde mit einer motorisierten Schlag-Schaum-Apparatur in Anlehnung an DIN 53902 bestimmt. Dazu wurden 340 ml einer Lösung hergestellt, die durch Verdünnen des wäßrigen Reinigungsmittels mit Leitungswasser aus Düsseldorf-Holthausen mit 18 ° dH derart hergestellt worden war, daß sie 2 Gew.-% Aktivsubstanz an Tensiden enthielt. Der Schaum wurde bei Raumtemperatur mit einer Lochplatte (Bohrungen von 1 mm Durchmesser, 10 Schläge bei einer Frequenz von 50 Schlägen/min, 13 cm Hub) erzeugt; er war sehr feinporig und entsprach somit weitgehend einem beim Shampoonieren auf dem Kopf entstehenden Schaum. Die Messungen wurden ohne Fettbelastung der Tensidlösung als Doppelbestimmung durchgeführt.

Als <u>Standard</u> wurde das Schaumverhalten einer als gut schäumend bekannten Mischung aus

(a) 42 Gewichtsprozent Texapon N 25 (= 12 % Aktivsubstanz),
(b) 10 Gewichtsprozent Dehyton K (= 3 % Aktivsubstanz),
(c) 1 Gewichtsprozent Comperlan LS (= 1 % Aktivsubstanz) und
(d) 47 Gewichtsprozent Wasser bestimmt. Diese Standard-Mischung wurde mit Wasser verdünnt und ebenfalls

in Form einer Lösung mit 2 Gew.-% Aktivsubstanz eingesetzt. Es wurden folgende Schaummengen gemessen:

- nach 1 Minute: 240 ml
- nach 3 Minuten: 210 ml
- nach 5 Minuten: 190 ml

Tabelle 4

| Relative Schaummenge | | | |
|---|---|---|---|
| Substanz | V7 | V8 | B4 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| POC | - | 1,0 | 1,0 |
| APG 600 | - | - | 2,0 |
| Wasser | ad 100 | ad 100 | ad 100 |
| Schaum 1' | 87 | 95 | 105 |
| Schaum 3' | 95 | 89 | 100 |
| Schaum 5' | 94 | 88 | 113 |

[1] Die Angaben im oberen Block der Tabelle beziehen sich auf Gew.-% Aktivsubstanz des wäßrigen Reinigungsmittels vor dem weiteren Verdünnen mit Wasser zu einer Lösung mit einem Gehalt an 2 % Aktivsubstanz an Tensiden. Die Angaben im unteren Block der Tabelle geben die prozentualle Schaummenge im Vergleich zum Standard an.

Das erfindungsgemäße Beispiel B4 zeigt eine im Vergleich zu V7 und V8 deutlich höhere Schaummenge.

## 6. Hautverträglichkeits-Untersuchungen

Zur Bestimmung der Hautverträglichkeit der Reinigungsmittel wurde die von Zeidler und Reese entwickelte in-vitro-Methode verwendet, die in der Zeitschrift Ärztliche Kosmetologie 13, 39-45 (1983) ausführlich dargestellt ist. Als Maß für die Hautverträglichkeit der Reinigungsmittel diente die Quellung von Schweine-Epidermis. Dazu wurde die benötigte Epidermis unmittelbar nach der Schlachtung junger Schweine gewonnen und tiefgekühlt gelagert.

Für die Messung wurden ausgestanzte Epidermisstreifen der Größe 1 cm x 6 cm 30 Minuten lang in eine Lösung getaucht, die durch Verdünnen des wäßrigen Reinigungsmittels mit Wasser derart hergestellt worden war, daß sie 2 Gew.-% Aktivsubstanz an Tensiden enthielt. Die Lösung war auf 39 °C temperiert und auf pH = 6,5 eingestellt. Sodann wurde nach kurzem Spülen und Entfernen des anhaftenden Wasser durch leichtes Abpressen unter definierten Bedingungen das Gewicht der gequollenen Streifen bestimmt. Anschließend wurden die Streifen 24 Stunden über Calciumchlorid entwässert und erneut gewogen. Um Einflüsse auszuschalten, die auf spezifische Eigenschaften des jeweiligen Tieres oder den Entnahmeort (Rücken, Seite) zurückgehen, wurde jeweils eine Standardmessung durchgeführt. Dabei wird ein unmittelbar benachbarter Epidermisstreifen in gleicher Weise mit Wasser anstelle des wäßrigen Reinigungsmittels behandelt.

Die Meßwerte t für die Tensid-Behandlung und w für die Behandlung mit Wasser ergeben sich aus der Beziehung:

$$t, w = \frac{\text{Gewicht(gequollene Epidermis) - Gewicht(trockene Epidermis)}}{\text{Gewicht(trockene Epidermis)}}$$

Die standardisierte, relative Quellungsänderung Q ist schließlich definiert als

$$Q = ( t/w - 1 ) * 100 \%$$

Der Q-Wert der wasserbehandelten Haut ist somit definitionsgemäß 0 %; negative Werte weisen auf quellungshemmende Eigenschaften hin.

Die Ergebnisse der Quellungsmessungen sind in der Tabelle 5 zusammengestellt. Es stellte sich heraus, daß das erfindungsgemäße Beispiel B5 im Vergleich zu V9 und V10 verbesserte Quellwerte aufweist.

Tabelle 5

| Quellungsmessungen[1] | | | |
|---|---|---|---|
| Substanz | V9 | V10 | B5 |
| N25 | 9,0 | 9,0 | 9,0 |
| AKYP | 3,0 | 3,0 | 3,0 |
| Dehyton K | 3,0 | 3,0 | 3,0 |
| Nutrilan I | 0,5 | 0,5 | 0,5 |
| POC | - | 1,0 | 1,0 |
| APG 600 | - | - | 2,0 |
| Wasser | ad 100 | ad 100 | ad 100 |
| Quellwert[2] | 50 | 44 | 24 |

[1] Die Angaben im oberen Block der Tabelle beziehen sich auf Gew.-% Aktivsubstanz des wäßrigen Reinigungsmittels vor dem weiteren Verdünnen mit Wasser zu einer Lösung mit einem Gehalt an 2 % Aktivsubstanz an Tensiden. Die Angaben im unteren Block der Tabelle beziehen sich auf den experimentell ermittelten Quellwert Q.
[2] Die Fehlergrenzen betrugen bei allen Versuchen ± 7.

**Patentansprüche**

1. Wäßrige Reinigungsmittel enthaltend

    (A) 1 bis 50 Gew.-% eines oder mehrerer Aniontenside, die 1 oder 2 lipophile Reste mit 1 bis 22 C-Atomen und einen polaren Rest ausgewählt aus der Gruppe der Carboxylat-, Sulfat- oder Sulfonatreste und gegebenenfalls einen Polyoxyalkylenrest mit einem mittleren Alkoxylierungsgrad von 1 bis 15 enthalten,
    (B) 0,5 bis 10 Gew.-% eines oder mehrerer Alkylglycoside der allgemeinen Formel $R(G)_x$ worin R einen linearen, gesättigten Alkylrest mit 8 bis 22 C-Atomen und $(G)_x$ ein Glycosid- oder Oligoglycosidrest mit einem Oligomerisationsgrad x von 1 bis 4 ist,
    (C) 0,1 bis 5 Gew.-% eines anionischen Polymers,
    (D) 35 bis 98,4 Gew.-% Wasser,

    wobei die Summe der Komponenten (B) und (C) und nicht größer als der Gehalt an Komponente (A) ist.

2. Wäßrige Zubereitungen nach Anspruch 1, worin der Anteil der Aniontenside (A) 5 bis 30 Gew.-% beträgt.

3. Wäßrige Zubereitungen nach Anspruch 1 oder 2, worin der Anteil des Alkylglykosids (B) 1 bis 5 Gew.-% beträgt.

4. Wäßrige Zubereitungen nach einem der Ansprüche 1 bis 3, worin der Anteil des anionischen Polymers (C) 0,5 bis 3 Gew.-% beträgt.

5. Wäßrige Zubereitungen nach einem der Ansprüche 1 bis 4, die zusätzlich 0,1 bis 10 Gew.-% eines anorganischen Elektrolytsalzes (E) enthalten.

**Claims**

1. Aqueous cleaning preparations containing

   (A) 1 to 50% by weight of one or more anionic surfactants containing 1 or 2 lipophilic groups with 1 to 22 carbon atoms and a polar group selected from carboxylate, sulfate or sulfonate groups and optionally a polyoxyalkylene group having an average degree of alkoxylation of 1 to 15,

   (B) 0.5 to 10% by weight of one or more alkyl glycosides corresponding to the general formula $R(G)_x$ in which R is a linear, saturated $C_{8-22}$ alkyl radical and $(G)_x$ is a glycoside or oligoglycoside having a degree of oligomerization x of 1 to 4,

   (C) 0.1 to 5% by weight of an anionic polymer,

   (D) 35 to 98.4% by weight of water,

   the sum total of components (B) and (C) being no greater than the content of component (A).

2. Water-containing preparations as claimed in claim 1 in which the percentage content of the anionic surfactants (A) is 5 to 30% by weight.

3. Water-containing preparations as claimed in claim 1 or 2, in which the percentage content of the alkyl glycoside (B) is 1 to 5% by weight.

4. Water-containing preparations as claimed in any of claims 1 to 3, in which the percentage content of the anionic polymer (C) is 0.5 to 3% by weight.

5. Water-containing preparations as claimed in any of claims 1 to 4 which additionally contain 0.1 to 10% by weight of an inorganic electrolyte salt (E).

**Revendications**

1. Détergents aqueux renfermant

   (A) 1 à 50 % en poids d'un ou de plusieurs surfactifs anioniques, qui possèdent 1 ou 2 radicaux lipophiles comportant 1 à 22 atomes de C et un radical polaire sélectionné parmi le groupe constitué des radicaux carboxylate, sulfate ou sulfonate et, le cas échéant, un radical polyoxyalkylène présentant un degré d'alcoxylation moyen compris entre 1 et 15,
   (B) 0,5 à 10 % en poids d'un ou de plusieurs alkylglycosides de la formule générale $R(G)_x$, dans laquelle R représente un radical alkyle linéaire saturé comportant 8 à 22 atomes de C, et $(G)_x$ correspond à un radical glycoside ou oligoglycoside présentant un degré d'oligomérisation x de 1 à 4,
   (C) 0,1 à 5 % en poids d'un polymère anionique,
   (D) 35 à 98,4 % en poids d'eau,

   la somme des composants (B) et (C) n'étant pas supérieure à la concentration en composant (A).

2. Préparations aqueuses selon la revendication 1, caractérisées en ce que la proportion des tensioactifs anioniques (A) atteint 5 à 30 % en poids.

3. Préparations aqueuses selon la revendication 1 ou 2, caractérisées en ce que la proportion de l'alkylglycoside (B) atteint 1 à 5 % en poids.

4. Préparations aqueuses selon la revendication 1 ou 2, caractérisées en ce que la proportion du polymère anionique (C) atteint 0,5 à 3 % en poids.

5. Préparations aqueuses selon une des revendications 1 à 4, caractérisées en ce qu'elles contiennent en plus 0,1 à 10 % en poids d'un sel électrolytique inorganique (E).